(19) Europäisches Patentamt
European Patent Office
Office européen des brevets

(11) Publication number: **0 170 093**
**B1**

# (12) EUROPEAN PATENT SPECIFICATION

(45) Date of publication of patent specification: **07.03.90**

(51) Int. Cl.[5]: **C 07 D 209/94,** A 61 K 31/40

(21) Application number: **85108276.8**

(22) Date of filing: **05.07.85**

## (54) 1,2,3,3a,8,8a-Hexahydro-Indeno-(1,2-c)Pyrrdes.

(30) Priority: **25.07.84 US 634235**

(43) Date of publication of application:
**05.02.86 Bulletin 86/06**

(45) Publication of the grant of the patent:
**07.03.90 Bulletin 90/10**

(84) Designated Contracting States:
**BE CH DE FR GB IT LI NL SE**

(56) References cited:
**JOURNAL OF PHARMACEUTICAL SCIENCES, vol. 57, no. 5, May 1968, pages 1013-1016, US; S.C. LAHIRI et al.: "Synthesis and pharmacology of some pyrroles and indan amines: hexahydro indeno (1,2-c) pyrroles and indan amines"**

(73) Proprietor: **ABBOTT LABORATORIES**
**14th Street and Sheridan Road North St**
**North Chicago Illinois 60064 (US)**

(72) Inventor: **DeBernardis, John F.**
**35 Burnett Avenue**
**Lake Villa Illinois (US)**
Inventor: **Winn, Martin**
**1263 Carlisle Place**
**Deefield Illinois (US)**

(74) Representative: **Modiano, Guido et al**
**MODIANO, JOSIF, PISANTY & STAUB Modiano & Associati Via Meravigli, 16**
**I-20123 Milano (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 170 093 B1

## Description

Technical Field

This invention relates to novel 1,2,3,3a,8,8a-hexahydro-indeno[1,2-c]pyrroles useful in the treatment of hypertension.

Background

The adrenergic nervous system plays a major role in the innervation of heart, blood vessel and smooth muscle tissue. Agents capable of interacting with receptor sites within the adrenergic nervous system can result in a variety of physiological responses, including vasoconstriction, vasodilation, and increased or decreased heart rate (chronotropic), contractility (inotropic) and metabolic activity. In the past, various adrenergic agents have been employed to affect these and other physiological responses. However, it is highly desirable to obtain new adrenergic agents which demonstrate a high degree of specificity for differing receptor types within the adrenergic nervous system in order to obtain a desired physiological response separate from other possible, and perhaps less desirable, responses of the system. This property has been lacking from most previously employed adrenergic agents. Thus, the search continues for new and improved adrenergic agents capable of selective interaction with adrenergic receptor sites.

It has now been determined that a new class of compounds, the 1,2,3,3a,8,8a-hexahydro-indeno[1,2-c]pyrroles, as herein defined, demonstrate an ability to interact specifically with various adrenergic receptor types and are useful in the treatment of hypertension.

Disclosure of the Invention

The present invention provides 1,2,3,3a,8,8a-hexahydro-indeno[1,2-c]pyrroles represented by the formula

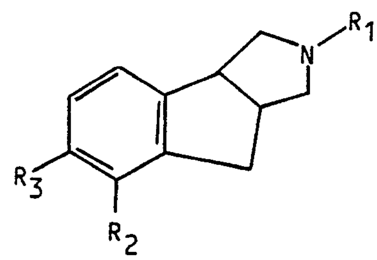

wherein $R_1$ is hydrogen, lower alkyl, lower acyl or benzyl; $R_2$ and $R_3$ may be the same or different and are hydroxy or lower alkoxy; or the compound is a pharmaceutically acceptable salt thereof. Preferred compounds of the present invention are those for which $R_1$ is hydrogen, methyl and ethyl and $R_2$ and $R_3$ are hydroxyl.

As used herein, the term "lower alkyl" refers to straight or branched chain saturated hydrocarbon radicals having 1 to 5 carbon atoms, such as methyl, ethyl, *n*-propyl, *iso*-propyl *n*-butyl, *s*-butyl, and *t*-butyl. The term includes halo-substituted lower alkyl radicals such as, for example, trifluoromethyl, 2-trichloroethyl.

As used herein, the term "halo" means chloro, bromo, fluoro and iodo.

As used herein, the term "lower alkoxy" means an alkoxy group represented by the formula —O—R wherein R is lower alkyl as herein defined.

As used herein, the term "lower acyl" means an acyl group represented by the formula

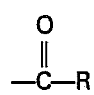

wherein R is lower alkyl as herein defined. Illustrative acyl groups useful in the practice of the invention are acetyl, *n*-propionyl, *n*-butyryl, *s*-butyryl, and *iso*-butyryl.

The term "pharmaceutical acceptable salts" refers to the pharmaceutically acceptable, relatively nontoxic, inorganic or organic acid addition salts of the compounds of this invention. These salts can be prepared *in situ* during the final isolation and purification of the compounds, or by separately reacting the free base with a suitable organic or inorganic acid. Representative salts include the hydrochloride, hydrobromide, sulfate, phosphate, nitrate, bisulfate, acetate, oxalate, valerate, oleate, palmitrate, stearate, laurate, borate, benzoate, lactate, phosphate, tosylate, citrate, maleate, fumarate, succinate, tartrate, and napsylate. It will be apparent to those skilled in the art that, depending upon the number of available amino groups for salt formation, the salt of this invention can be per-*N*-salts.

The foregoing may be better understood in connection with the following examples:

Example 1

4,5-Dimethoxy indane, 1,2-dicarboxylic acid diethyl ester

88.9 g 4,5-dimethoxy indene, 1,2-dicarboxylic acid, diethyl ester (J.A.C.S. *73* 5826, 1956) in 1 liter ethanol was hydrogenated over 120 g. Raney Nickel catalyst at 3 atmospheres pressure. The catalyst was filtered and the solution concentrated to give 85 g product as an oil.

Example 2

4,5-Dimethoxy indane, 1,2-dicarboxylic acid

45.8 g of the diethyl ester of Example 1, 60 ml ethanol, 90 ml water, and 69 g 45% potassium hydroxide (KOH) were refluxed 1 hour. The solution was concentrated in vacuum and acidified with hydrochloric acid. The resulting solid was filtered and dissolved in dimethoxyethane. The solution was dried over magnesium sulfate ($MgSO_4$), concentrated and benzene added, to get the desired product, 35.06 g, mp.p. 153—160°C.

Analysis, theoretical: C, 58.64; H, 5.30.
Found: C, 58.40; H, 5.39.

Example 3

4,5-Dimethoxy indane, 1,2-dicarboxylic acid anhydride

5.00 g of the dicarboxylic acid of Example 2, 50 ml acetic anhydride and 50 mg toluene sulfonic acid monohydrate were refluxed for 1 hour. The acetic anhydride was removed on the rotovac and the residue distilled to give 4.13 g product, b.p. 190—195°C/0.3 mm Hg ($4 \times 10^{-4}$).

Example 4

2-Benzyl-6,7-dimethoxy-1,2,3,3a,8,8a-hexahydro-indeno[1,2-c]pyrrole-1,3-dione

4.00 g of the resultant anhydride of Example 3 was suspended in 80 ml xylene, then 1.90 g benzylamine was added. The mixture was refluxed 3 hours. Benzene (60 ml) was added to the cooled solution and a small amount of solid was filtered. The benzene filtrate was washed with dilute hydrochloric acid and then with dilute potassium bicarbonate. After drying ($MgSO_4$), and concentrating, ether was added to get 4.50 g product, m.p. 130—132°C (83% yield).

Analysis, theoretical: C, 71.20; H, 5.68; N, 4.15.
Found: C, 71.01; H, 5.62; N, 4.21.

Example 5

2-Benzyl-6,7-dimethoxy-1,2,3,3a,8,8a-hexahydro-indeno[1,2-c]pyrrole hydrochloride

6.19 g of the resultant compound of Example 4 suspended in 15 ml tetrahydrofuran (THF) was treated with 100 ml 1M $BH_3$ in THF under nitrogen atmosphere. The solution was refluxed 4 hours, then cooled and a solution of 35 ml concentrated hydrochloric acid and 35 ml water was added slowly. The solution was refluxed for 20 minutes and then concentrated in vacuum. The residue was made basic with potassium hydroxide solution and extracted with chloroform. The chloroform extracts were dried ($K_2CO_3$) and concentrated. The residue was acidified with HCl in isopropyl alcohol and this solution was concentrated. Acetonitrile was added to give the product 5.78 g., m.p. 176—178°C.

Analysis, theoretical: C, 69.40; H, 6.99; N, 4.05.
Found: C, 69.01; H, 7.03; N, 3.98.

Example 6

6,7-Dimethoxy-1,2,3,3a,8,8a-hexahydro-indeno[1,2-c]pyrrole hydrochloride

4.36 g of the resultant compound of Example 5 was hydrogenated with 0.9 g 5% Pd/C in 100 ml methanol at 3 atmospheres (3 bar), affording 2.95 g of product (92%), m.p. 176—178°C.

Analysis, theoretical with ¼ $H_2O$): C, 60.22; H, 6.80; N, 5.40.
Found: C, 60.00; H, 6.91; N, 5.42.

Example 7

1,2,3,3a,8,8a-Hexahydro-indeno[1,2-c]pyrrole 6,7-diol hydrobromide

2.00 g of the resultant compound of Example 6 in 13 ml $CH_2Cl_2$ under a nitrogen atmosphere at −78°C was treated with 10 g boron tribromide ($BBr_3$). The mixture was stirred at 0°C for 1 hour and then cooled to −78°C while 50 ml methanol was added slowly. The solution was concentrated in vacuum and the residue was crystallized from acetonitrile to get 1.97 g product, m.p. 206—208°C.

Analysis, theoretical: C, 48.55; H, 5.18; N, 5.15.
Found: C, 48.26; H, 5.16; N, 5.11.

Example 8

6,7-Dimethoxy-2-methyl-1,2,3,3a,8,8a-hexahydro-indeno[1,2-c]pyrrole hydrochloride

1.50 g of the resultant compound of Example 6, 0.15 g 5% Pd/C in 95 ml methanol, 5 ml 37% formaldehyde and 0.8 g sodium acetate were hydrogenated at 3 atmospheres (3 bar). The solution was concentrated after removal of the catalyst and the residue was made basic with KOH in water. This was extracted with $CHCl_3$, dried ($K_2CO_3$) and concentrated. The residue was dissolved in acetonitrile and

acidified with hydrochloric acid in ether to get 1.40 g product, m.p. 160—162°C

Analysis, theoretical: C, 62.33; H, 7.47; N, 5.19.
Found: C, 62.27; H, 7.49; N, 5.28.

Example 9

2-Methyl-1,2,3,3a,8,8a-hexahydro-indeno[1,2-c]pyrrole-6,7-diol hydrobromide

1.81 g of the resultant compound of Example 8 was treated with $BBr_3$ as described in Example 7 to get 1.85 g product, m.p. 191—193°C.

Analysis, theoretical (with ¼ water: C, 49.58; H, 5.72; N, 4.82.
Found: C, 49.78; H, 5.63; N, 4.76.

Example 10

2-Acetyl-6,7-dimethoxy-1,2,3,3a,8,8a-hexahydro-indeno[1,2-c]pyrrole

1.36 g of the resultant compound of Example 6 was basified with KOH in water and extracted into ether. The ether was evaporated and the residue dissolved in 5 ml $CHCl_3$ and treated with 1.7 ml. acetic anhydride. After refluxing for 10 minutes, the solution was concentrated and the excess acetic anhydride decomposed with KOH in water. The residue was extracted with $CHCl_3$, dried ($MgSO_4$) and concentrated to give 1.5 g product which was used directly in the next step (Example 11).

Example 11

2-Ethyl-6,7-dimethoxy-1,2,3,3a,8,8a-hexahydro-indeno[1,2-c]pyrrole hydrochloride

The resultant compound of Example 10 was dissolved in 15 ml THF and this solution was added to a suspension of 0.6 g $LiAlH_4$ in 8 ml THF. The reaction mixture was refluxed 3 hours and worked up with 1 ml water and 1.5 ml 25% NaOH. Concentrating the THF solution yielded the amine as an oil. The hydrochloride was made with hydrochloric acid in $CH_2Cl_2$ and was used without further purification.

Example 12

2-Ethyl-1,2,3,3a,8,8a-hexahydro-indeno[1,2-c]pyrrole hydrobromide

The product of Example 11 was treated with $BBr_3$ as described in Example 7 to give 620 mg product, m.p. 195—198°C.

Analysis, theoretical (with ⅓ water: C, 59.65; H, 7.15; N, 5.35.
Found: C, 59.67; H, 7.15; N, 5.29.

Example 13

2-Propyl-1,2,3,3a,8,8a-hexahydro-indeno[1,2-c]pyrrole-6,7-diol hydrobromide

The compound of Example 6 (4.00 g) was converted in 92% yield to the N-propionyl amide by the method of Example 10. This was reduced to the amine in 65% yield by the method in Example 11 and this was demethylated with $BBr_3$ by the method of Example 7 in 80% yield to give the desired product, m.p. 192—195°C.

Analysis, theoretical: C; 53.50; H, 6.42; N, 4.46.
Found: C, 53.14; H, 6.36; N, 4.44.

The therapeutic activity of the compounds can be demonstrated in vivo by their ability to decrease arterial blood pressure and/or heart rate in the spontaneously hypertensive rat as follows: A group of Okamoto rats, which develop hypertension spontaneously when reaching young adulthood, are deprived of food for a period of 16 hours and are placed in semi-restraining wire mesh cylinders maintained at a constant temperature of 35°C. An occluding cuff, operatively connected to a programmed sphygmomanometer, is placed over the tail of each rat of the group and retained near the tail base. The pressure of each cuff is automatically, cylically increased within the range of from 0 to 250 mm Hg (0.33 bar) at the rate of 10mm Hg (0.013 bar)/sec, the total inflation and deflation time of each cycle being 50 seconds, with a 10 second rest period between cycles. A photocell is placed distal to the cuff to detect pulses resulting from the forward motion of blood flow with each heartbeat of the rat. As the pressure in the cuff increases, measurable pulses disappear at the point where the cuff pressure equals the arterial blood pressure. Measurable pulses reappear during deflation at approximately the same pressure, and arterial blood pressure is thereby established by cuff pressure at the point of pulse appearance. The heart rate is determined from the arterial pulse wave. A 100 mg/kg dose of a test compound of Formula I is administered orally to each rat of the test group, and five interference-free signals are recorded on a polygraph for each rat at various measurement periods following administration. By following the foregoing procedure, the tested preferred compounds of the invention are shown to decrease the arterial blood pressure and/or heart rate of each rats of the group.

The compounds of the invention can be administered in any effective pharmeutically acceptable form to warm blooded animal, e.g., in oral parenteral or infusable dosage forms, or a buccal or nasal spray. Suitable parenteral routes of administration include, for example, intramuscular, intravenous, intraperitoneal or subcutaneous administration of the compounds.

In addition to the active compounds, compositions according to this invention for parenteral injection may comprise pharmaceutically acceptable sterile aqueous or nonaqueous solutions, suspensions or

emulsions. Examples of suitable nonaqueous carriers, diluents, solvents or vehicles include propylene glycol, polyethylene glycol, vegetable oils, such as olive oil, and injectable organic esters such as ethyl oleate. Such compositions may also contain adjuvants such as preserving, wetting, emulsifying and dispersing agents. They may be sterilized, for example, by filtration through a bacteria-retaining filter, or by incorporating sterilizing agents into the composition. They can also be manufactures in the form of sterile solid compositions which can be dissolved in sterile water, or other sterile injectable medium, immediately before use.

Solid dosage forms for oral administration include capsules, tablets, pills, powders and granules. In such solid dosage forms, the active compound may be admixed with at least one inert diluents such as sucrose, lactose or starch. Such dosage forms may also comprise, as in normal practice, additional substances other than inert diluents, e.g., lubricating agents such as magnesium stearate. In the case of capsules, tablets and pills, the dosage forms may also comprise buffering agents. Tablets and pills can additionally be prepared with enteric coatings.

Liquid dosage forms for oral administration include pharmaceutically acceptable emulsions, solutions, suspensions, syrups and elixirs containing inert diluents commonly used in the art, such as water. Besides such inert diluents, compositions may also comprise adjuvants, such as wetting agents, emulsifying and suspending agents, and sweetening, flavoring and perfuming agents.

Actual dosage levels of active ingredients in the compositions of the invention may be varied so as to obtain an amount of active ingredients effective to obtain a desired therapeutic response for a particular composition and method of administration. The selected dosage level therefore depends upon the desired therapeutic effect, on the route of administration, on the desired duration of treatment and other factors. Generally, dosage levels of about 0.1 to about 200, more preferably about 0.5 to about 150 and most preferably about 1 to about 125 mg of active ingredients per kg of body weight per day are administered orally to a mammalian patient suffering from hypertension. If desired, the daily dose may be divided into multiple doses for administration, e.g., two to four separate doses per day.

**Claims**

1. A compound of the formula

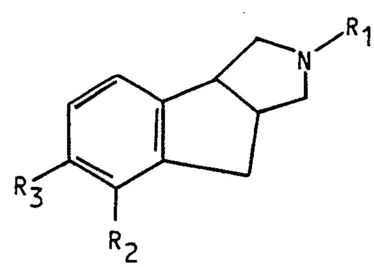

wherein $R_1$ is hydrogen, lower alkyl with 1 to 5 carbon atoms, and such lower alkyls substituted by halo groups, lower acyl with 1 to 5 carbon atoms or benzyl; $R_2$ and $R_3$ may be the same or different and are hydroxy or lower alkoxy with 1 to 5 carbon atoms or pharmaceutically acceptable salt thereof.

2. The compound of Claim 1 wherein $R_1$ is hydrogen and $R_2$ and $R_3$ are hydroxyl.

3. The compound of Claim 1 wherein $R_1$ is ethyl and $R_2$ and $R_3$ are hydroxyl.

4. The compound of Claim 1 wherein $R_1$ is methyl and $R_2$ and $R_3$ are hydroxyl.

5. The compound of Claim 1, 1,2,3,3a,8,8a-hexahydro-indeno[1,2-c]pyrrole-6,7-diol hydrobromide.

6. The compound of Claim 1, 2-methyl-1,2,3,3a,8,8a-hexahydro-indeno[1,2-c]pyrrole-6,7-diol hydrobromide.

7. The compound of Claim 1, 2-ethyl-1,2,3,3a,8,8a-hexahydro-indeno[1,2-c]pyrrole-6,7-diol hydrobromide.

8. A pharmaceutical composition comprising a therapeutically effective amount of the compound, according to any one of claims 1 to 7.

9. Used of a compound according to any one of claims 1 to 7 for preparing a drug for treating hypertension.

**Patentansprüche**

1. Verbindung der Formel

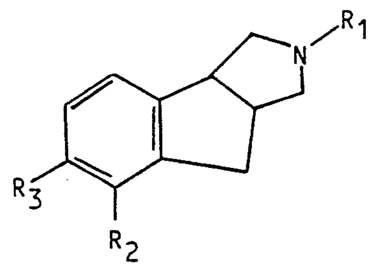

worin Wasserstoff, Niederalkyl mit 1 bis 5 Kohlenstoffatomen und solche Niederalkyle, die durch Halogengruppen substituiert sind, Niederacyl mit 1 bis 5 Kohlenstoffatomen oder Benzyl ist; $R_2$ und $R_3$ gleich oder verschieden sein können und Hydroxy oder Niederalkoxy mit 1 bis 5 Kohlenstoffatomen sind, oder ein pharmazeutisch annehmbares Salz davon.

2. Verbindung nach Anspruch 1, worin $R_2$ Wasserstoff ist und $R_2$ und $R_3$ Hydroxyl sind.

3. Verbindung nach Anspruch 1, worin $R_2$ Ethyl ist und $R_2$ und $R_3$ Hydroxyl sind.

4. Verbindung nach Anspruch 1, worin $R_2$ Methyl ist und $R_2$ und $R_3$ Hydroxyl sind.

5. Verbindung nach Anspruch 1, 1,2,3,3a,8,8a-Hexahydroindeno[1,2-c]pyrrole-6,7-diol-hydrobromid.

6. Verbindung nach Anspruch 1, 2-Methyl-1,2,3,3a,8,8a-Hexahydroindeno[1,2-c]pyrrole-6,7-diol-hydrobromid.

7. Verbindung nach Anspruch 1, 2-Ethyl-1,2,3,3a,8,8a-Hexahydroindeno[1,2-c]pyrrole-6,7-diol-hydrobromid.

8. Pharmazeutische Zusammensetzung, welche eine therapeutisch wirksame Menge der Verbindung nach einem der Ansprüche 1 bis 7 umfaßt.

9. Verwendung einer Verbindung nah einem der Ansprüche 1 bis 7 zur Herstellung eines Medikaments zur Behandlung von Bluthochdruck.

**Revendications**

1. Un composé de formule

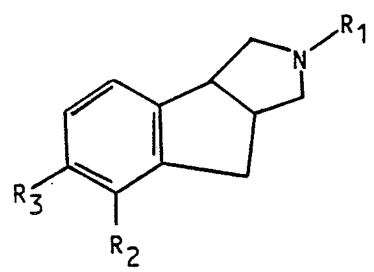

dans laquelle $R_1$ est un atome d'hydrogène, un groupe alkyle inférieur de 1 à 5 atomes de carbone, un groupe alkyle inférieur de ce type substitué par des groupes halo, un groupe acyle inférieur de 1 à 5 atomes de carbone ou un groupe benzyle; $R_2$ et $R_3$ peuvent être identiques ou différents et représentent un groupe hydroxy ou un groupe alcoxy inférieur de 1 à 5 atomes de carbone ou un sel pharmaceutiquement acceptable dudit composé.

2. Le composé selon la revendication 1, selon lequel $R_1$ est un atome d'hydrogène et $R_2$ et $R_3$ sont des groupes hydroxyles.

3. Le composé selon la revendication 1, selon lequel $R_1$ est un groupe éthyle et $R_2$ et $R_3$ sont des groupes hydroxyles.

4. Le composé selon la revendication 1, selon lequel $R_1$ est un groupe méthyle et $R_2$ et $R_3$ sont des groupes hydroxyles.

5. Le composé selon la revendication 1, que est le bromhydrate de 1,2,3,3a,8,8a-hexahydro-indéno[1,2-c]pyrrole-6,7-diol.

6. Le composé selon la revendication 1, que est le bromhydrate de 2-méthyl-1,2,3,3a,8,8a-hexahydro-indéno[1,2-c]pyrrole-6,7-diol.

7. Le composé selon la revendication 1, que est le bromohydrate de 2-éthyl-1,1,3,3a,8,8a-hexahydro-indéno[1,2-c]pyrrole-6,7-diol.

8. Une composition pharmaceutique comprenant une quantité thérapeutiquement efficace du composé selon l'une quelconque des revendications 1 à 7.

9. Utilisation d'un composé selon l'une quelconque des revendications 1 à 7 pour préparer un médicament pour le traitement de l'hypertension.